# EUROPEAN PATENT APPLICATION

(11) **EP 4 817 668 A1**
(43) Date of publication of application: **07.10.2026**
(21) Application number: 26169929.2
(22) Date of filing: 31.03.2026
(51) Int. Cl.: A61M 25/09

(54) **MEDICAL GUIDEWIRE DEVICES**

(30) Priority: 05.04.2025 US 202519171194
(71) Applicant: Deepin Technologies, LLC, San Jose, CA 95131 (US)
(72) Inventor: SCHABERT, Jon, San Jose, CA 95131 (US); WANG, Jiaxu, San Jose, CA 95131 (US); HUYNH, Erik, San Jose, CA 95131 (US); KUBACKI, Alex, San Jose, CA 95131 (US); DINH, Christopher, San Jose, CA 95131 (US)
(74) Representative: Knöner, Gregor

(57) **Abstract**

A guidewire device (100) is provided. The guidewire device (100) comprises: a core wire (110) extending between a proximal portion (112) and a distal portion (114); a tube member (150) located near the distal portion (114) of the core wire (110), the tube member (150) being secured to the core wire (110) and defining a space (120) between the core wire (110) and the tube member (150); and a plurality of disks (190) coupled to the distal portion (114) of the core wire, the plurality of disks (190) being spaced apart from each other and configured to interference-fit onto an inner surface (152) of the tube member (150), wherein the plurality of disks (190) each comprises an opening (192) configured to allow the core wire (110) to pass through and align substantially with a central longitudinal axis (151) of the tube member (150).

## Description

### TECHNICAL FIELD

This application relates generally to medical devices and methods of making and using medical devices to treat diseases. Particularly, various embodiments of guidewire devices and methods are described.

### BACKGROUND

Guidewire devices are widely used in the medical field for guiding an ancillary device to a particular location in a patient's body to perform delicate procedures e.g., guiding a catheter deep in the vasculature of the body. Guidewire devices often require a variable stiffness profile, typically with the most flexible section at the distal end while maintaining good torque transmission for trackability and delivery in tortuous anatomy.

A guidewire device generally includes a core wire, which may have a tapered distal section reinforced with a structure joined to an atraumatic tip. Traditionally, metal coils or braids are used as guidewire reinforcement. As micro-machining and laser cutting technologies have evolved, slotted hypotubes have also entered the field as device components.

While advancement has been made in the field of guidewire devices, need for improvement still exists. There is a need for centering a core wire within a hypotube-based guidewire device to enhance torque transmission and avoid or minimize kinking or whipping. There is a need for improving shapeability and shape retention property of a hypo tube-based guidewire to facilitate navigation through tortuous and complex pathways. It would be desirable to offer more options of materials for constructing core wires with various support or stiffness profiles. It would be desirable to provide the user of a guidewire with a visual indication of changes of stiffness profile of the guidewire to help delivery of ancillary devices.

### SUMMARY

In one aspect, embodiments of the disclosure feature a guidewire device. In general, an embodiment of the guidewire device comprises a core wire extending between a proximal portion and a distal portion and a tube member located near the distal portion of the core wire. The tube member is secured to the core wire and defines a space between the core wire and the tube member. An expandable structure is disposed in the space between the core wire and the tube member. The expandable structure is configured to interference-fit onto the inner surface of the tube member and comprises a proximal end having an opening and a distal end having an opening to allow the core wire to pass through the expandable structure. The opening of the proximal end and the opening of the distal end of the expandable structure substantially align with the central longitudinal axis of the tube member and are configured to encircle the core wire to thereby align the core wire substantially with the central longitudinal axis of the tube member.

In another aspect, embodiments of the disclosure feature a guidewire device. In general, an embodiment of the guidewire device comprises a core wire extending between a proximal portion and a distal portion and a tube member located near the distal portion of the core wire. The tube member is secured to the core wire and defines a space between the core wire and the tube member. A plurality of disks are coupled to the distal portion of the core wire. The plurality of disks are spaced apart from each other and configured to interference-fit onto the inner surface of the tube member. The plurality of disks each comprises an opening configured to allow the core wire to pass through and align substantially with a central longitudinal axis of the tube member.

In another aspect, embodiments of the disclosure feature a guidewire device. In general, an embodiment of the guidewire device comprises a core wire extending between a proximal portion and a distal portion, a tube member located near the distal portion of the core wire and coupled to the core wire, and a radiopaque marker in the tube member and coupled to the core wire, wherein the radiopaque marker comprises a radiopaque first material and a plastically deformable second material.

In a further aspect, embodiments of the disclosure feature a guidewire device. In general, an embodiment of the guidewire device comprises a core wire and an indicator secured to the core wire. The distal portion of the core wire comprises a first section having a first stiffness profile and a second section having a second stiffness profile different from the first stiffness profile. The indicator is located at the joint of the first section and the second section to provide a visual indication of a change of stiffness profile of the core wire.

In a further aspect, embodiments of the disclosure feature a guidewire device. In general, an embodiment of the guidewire device comprises a core wire extending between a proximal portion and a distal portion. The core wire comprises a drawn-filled tubing (DFT) wire comprising an inner core of a first material and an outer sheath of a second material different from the first material.

This Summary is provided to introduce selected aspects and embodiments of this disclosure in a simplified form and is not intended to identify key features or essential characteristics of the claimed subject matter, nor is it intended to be used as an aid in determining the scope of the claimed subject matter. The selected aspects and embodiments are presented merely to provide the reader with a summary of certain forms the invention might take and are not intended to limit the scope of the invention. Other aspects and embodiments of the disclosure are described in the section of Detailed Description.

These and various other aspects, embodiments, features, and advantages of the disclosure will become better understood upon reading of the following detailed description in conjunction with the accompanying drawings.

### BRIEF DESCRIPTION OF THE DRAWINGS

FIG. 1 is a simplified illustration of an example guidewire device according to embodiments of the disclosure.
FIG. 2 is a simplified illustration of the guidewire device of FIG. 1 with components separated to show the core wire, the tube member, and other components with greater clarity.
FIG. 3 is a cross-sectional end view of the guidewire device shown in FIG. 1, taken along lines A-A.
FIG. 4 schematically shows the use of a stent-like structure to center a core wire in a guidewire device according to embodiments of the disclosure.
FIG. 5 depicts a cut-tube structure which can be used to center a core wire in a guidewire device according to embodiments of the disclosure.
FIG. 6 depicts an expanded state of the cut-tube structure shown in FIG. 5 according to embodiments of the disclosure.
FIG. 7 depicts a spike crown-like structure which can be used to center a core wire in a guidewire device according to embodiments of the disclosure.
FIG. 8 depicts an expanded state of the spike crown-like structure shown in FIG. 7 according to embodiments of the disclosure.
FIG. 9 schematically shows the use of a plurality of disk-like structures to center a core wire in a guidewire device according to embodiments of the disclosure.
FIG. 10 is a cross-sectional end view of the guidewire device shown in FIG. 9, taken along lines B-B.
FIG. 11 depicts an example disk-like structure which can be used to center a core wire in a guidewire device according to embodiments of the disclosure.
FIG. 12 depicts another disk-like structure which can be used to center a core wire in a guidewire device according to embodiments of the disclosure.
FIG. 13 is a simplified illustration of an example guidewire device according to embodiments of the disclosure.
FIG. 14 is a simplified illustration of an example core wire including a radiopaque marker coupled to the distal end portion of the core wire according to embodiments of the disclosure.
FIG. 15 depicts an example radiopaque coil according to embodiments of the disclosure.
FIG. 16 depicts an example radiopaque coil according to alternative embodiments of the disclosure.
FIG. 17 depicts an example radiopaque coil according to alternative embodiments of the disclosure.
FIG. 18 depicts an example radiopaque braid according to embodiments of the disclosure.
FIG. 19 is a simplified illustration of an example guidewire device according to embodiments of the disclosure.
FIG. 20 is a simplified illustration of the guidewire device shown in FIG. 19, with the tube member being removed to show the core wire and other components with greater clarity.
FIG. 21 is a cross-sectional end view of the example guidewire device shown in FIG. 3-1, taken along lines C-C.
FIG. 22 is a simplified illustration of an example core wire according to embodiments of the disclosure.
FIGS. 23A, 23B, and 23C are simplified illustrations showing cross-sectional end views of example core wires according to embodiments of the disclosure.

### DETAILED DESCRIPTION OF EMBODIMENTS

With reference to the figures, various embodiments of guidewire devices and methods will now be described. The figures are intended to facilitate description of embodiments of the disclosure and are not necessarily drawn to scale. Certain specific details may be set forth in the figures to provide a thorough understanding of the disclosure. It will be apparent to one of ordinary skill in the art that some of these specific details may not be employed to practice embodiments of the disclosure. In other instances, structures, components, systems, materials, and/or operations often associated with known medical procedures may not be shown or described in detail to avoid unnecessarily obscuring description of embodiments of the disclosure.

The disclosure provides guidewire devices comprising unique features that can enhance the performance of the devices. Embodiments of the disclosure use metal composite wires to construct core wires, allowing for more options of materials through various combinations of the metal composite wire inner core and outer sheath, and/or through adjusting the metal composite wire inner core fill percentage. A radiopaque coil or braid comprising two or more different materials can be coupled to the distal end of a core wire to improve the shapeability and shape retention of the guidewire device. An indicator can be attached to a core wire to provide a visual indication of changes of the stiffness profile of the core wire to assist the physician during the clinical use. Embodiments of the disclosure also provide methods of centering a core wire in a hypotube-based guidewire device to improve torque transmission control and other performance.

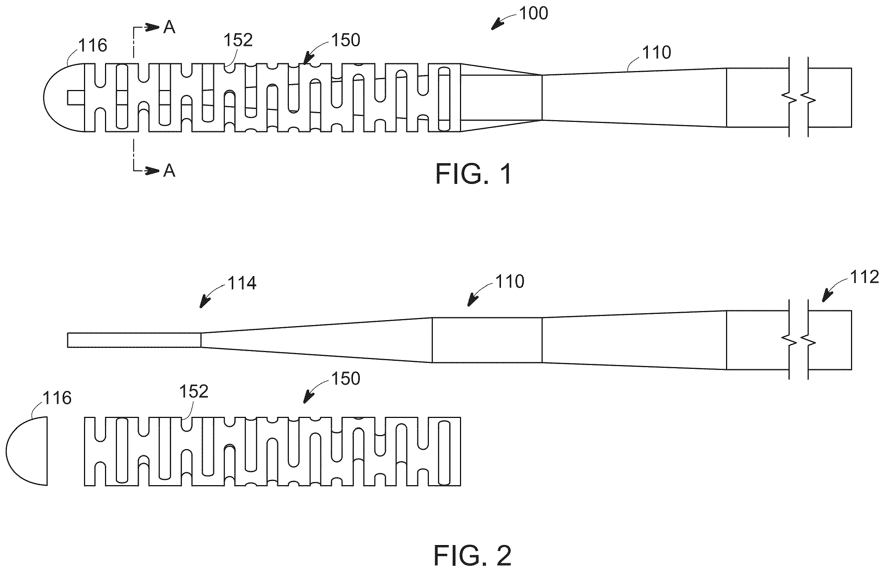

FIGS. 1-2 schematically illustrate an example guidewire device 100 according to embodiments of the disclosure. The guidewire device 100 is generally configured for use in conjunction with a medical device to perform procedures such as neuro-, cardio-, or peripheral vasculature interventions. One example application of the guidewire device 100 of the disclosure is for guiding a catheter deep within the neuro vasculature. In a broad overview, the guidewire device 100 includes an elongate core wire 110 and a tube member 150 coupled to the core wire 110. The core wire 110 extends between a proximal portion 112 and a distal portion 114, and has a length suitable for a particular application. The distal portion 114 of the core wire 110 may be tapered towards the distal end to provide more bending flexibility. The proximal portion 112 of the core wire 110 may have an increased diameter to maintain pushability and torsional rigidity of the guidewire device 100. The tube member 150 may be located near the distal portion 114 of the core wire 110 and secured to the core wire 110 to provide reinforcement and improve performance of the guidewire device 100. The tube member 150 can be secured to the distal portion 114 of the core wire 110 via various means e.g., bonding, welding, soldering, etc. to allow transmission of torsional force from the proximal section 112 of the core wire 110 to the tube member 150 and/or from the tube member 150 to the distal section 114 of the core wire 110. In the space defined between the tube member 150 and the distal portion 114 of the core wire 110, various components such as a radiopaque marker, a centering device, a core wire stiffness indicator etc. (not shown in FIGS. 1-2) can be provided to perform various functions, as will be described in greater detail below. The tube member 150 can be a hypotube constructed from a shape-memory material, and may include a plurality of cuts 152 configured to improve the effectiveness of the guidewire device 100, e.g., providing a desirable balance between bending flexibility, torsional rigidity, tensile strength, etc. The plurality of cuts 152 may be vertical cuts and/or helical cuts circumferentially extending around the central longitudinal axis of the tube member. U.S. Ser. No. 18/963, 683 filed November 28, 2025 entitled "Guidewire and Medical Device including Laser Cut Tube" and U.S. Ser. No. 19/043,429 filed February 1, 2025 entitled "Intravascular Medical Devices Including Laser Cut Tube" describe various embodiments of cut-tube structures and can be used as the tube member of the guidewire device. Alternatively, a polymer jacket may be used as a reinforcement structure in place of the tube member 150. An atraumatic tip 116 e.g., in a rounded shape can be formed at the distal end of the guidewire device 100 to prevent damage to the vessel.

### CORE WIRE CENTERING

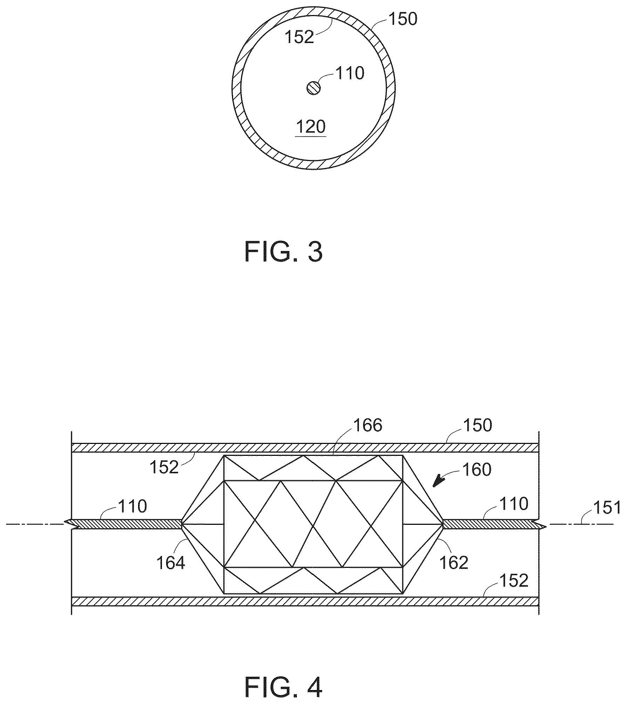

With reference to FIGS. 3-12, various embodiments of devices and methods for centering a core wire in a guidewire device are now described.

FIG. 3 is a simplified illustration of a cross-sectional end view of the guidewire device 100 of FIG. 1, showing a core wire 110, a tube member 150 surrounding the core wire 110, and a space 120 defined between the tube member 150 and the core wire 110 at a distal portion 114 of the guidewire device 100. The space 120 between the tube member 150 and the core wire 110 at the distal portion 114 of the guidewire device 100 may increase as the guidewire size increases e.g., from 0.254 mm to 0.356 mm (0.010 inches to 0.014 inches), 0.254 mm to 0.457 mm (0.010 inches to 0.018 inches), 0.254 mm to 0.610 mm (0.010 inches to 0.024 inches), and 0.254 mm to 0.965 mm (0.010 inches to 0.038 inches), and so on.

One issue associated with conventional hypotube-based guidewire devices is the misalignment of the core wire with the central longitudinal axis of the hypotube, i.e., the core wire is radially off-centered, especially at the distal portion of the guidewire device. Misalignment of the core wire can cause reduced torque transmission control, unstable tip behavior, among other issues. The problems can be exacerbated when the guidewire navigates a tortuous vessel with complex curves where the relatively stiffer core wire would not bend as much as the relatively more flexible hypotube. Severe misalignment of the core wire can cause whipping of the guidewire tip and loss of torque control, which would cancel out a purported benefit of hypotube-based guidewires - fine-tuned torque control of the guidewire tip for navigating through selected anatomies.

Conventional methods of centering a core wire use a coil or multiple coils to take up the free space between the core wire and the hypotube. In conventional methods, the centering coils do not provide functionalities other than merely filling the space between the core wire and the hypotube. Furthermore, conventional solutions cannot be easily scaled up because redesigning of centering coils would be required as the space between the core wire and the hypotube becomes larger in large guidewire devices.

According to embodiments of the disclosure, an expandable structure is used to center the core wire in the tube member of a guidewire device, or to align the core wire with the central longitudinal axis of the tube member. In general, the expandable structure is configured to interference-fit onto the inner surface of the tube member. The expandable structure comprises a proximal end having an opening and/or a distal end having an opening to allow the core wire to pass through the expandable structure. The opening of the proximal end and/or the opening of the distal end of the expandable structure substantially align with the central longitudinal axis of the tube member and are sized or configured to encircle the core wire to thereby align the core wire substantially with the central longitudinal axis of the tube member.

With reference to FIG. 4, according to embodiments of the disclosure the expandable structure 160 for centering the core wire 110 comprises a stent-like structure 160 e.g., having a plurality of cells with various shapes and sizes. The stent-like structure 160 comprises a collapsed state and an expanded state. The stent-like structure 160 comprises a proximal end portion 162, a distal end portion 164, and a main body portion 166. Each of the proximal end portion 162 and the distal end portion 164 may be tapered and have an opening sized or configured to allow the core wire 110 passing through. The main body portion 166 may have a pre-defined expanded configuration such as in a cylindrical shape which can interference-fit on the internal surface 152 of the tube member 150 and exert an outwardly radial force against the tube member 150. The openings in the tapered proximal end portion 162 and distal end portion 164 can be similar to or slightly greater than the cross-sectional dimension of the core wire 110 and align each other substantially on the central longitudinal axis 151 of the tube member 150, allowing the core wire 110 to be radially self-centered within the tube member 150. In alternative embodiments, the tapered proximal end portion 162 and/or distal end portion 164 can be secured to the core wire 110 via any suitable means such as bonding, welding, soldering, crimping, or the like.

The stent-like structure 160 can be braided using two or more filaments such as nitinol filaments or other metallic or polymeric shape-memory materials. Alternatively, the stent-like structure 160 can be formed by creating a plurality of fenestrations in a tube member of a shape-memory material using laser, blade, or other suitable means. Shape-memory materials tend to have a temperature induced phase change, causing the material to have a preferred configuration or shape which can be set by heating the material above a certain transition temperature. The stent-like structure 160 "remembers" the shape set during the heat treatment and tends to assume that shape when the structure is placed above the transition temperature. According to embodiments of the disclosure, the transition temperature can be selected to be higher than the room temperature but less than the body temperature for ease of assembly. This allows the stent-like structure 160 to only expand and create an interference fit with the tube member 150 once it is introduced into the body. Suitable metallic shape-memory materials for constructing the stent-like structure 160 include but are not limited to alloys of nickel-titanium (NiTi) or Nitinol^{®}, CuZnAl, FeNiAl, and so on. Suitable polymeric shape-memory materials for constructing the stent-like structure 160 include but are not limited to polytetrafluoroethylene (PTFE), polylactide (PLA), ethylene-vinyl acetate (EVA), and so on.

According to embodiments of the disclosure, one or more stent-like structures 160 may be used to center the core wire 110 based on the size, design, or application of the guidewire device 100.

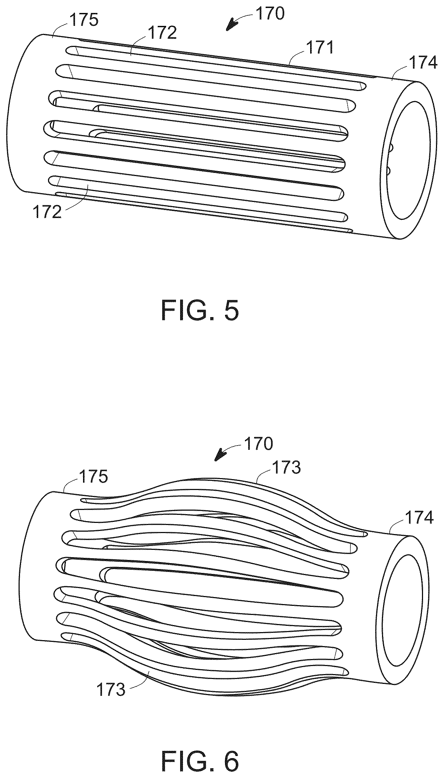

FIGS. 5-6 illustrates another example expandable structure 170 according to alternative embodiments of the disclosure. The expandable structure shown 170 in FIGS. 5-6 comprises a tubular body 171 of a shape-memory alloy or polymer. The tubular body 171 is provided with a plurality of longitudinal slits or cuts 172 forming a plurality of strands 173 extending between a proximal end portion 174 and a distal end portion 175 of the tubular body 171. The cut-tube structure 170 can have a non-expanded state (FIG. 5) and an expanded state (FIG. 6). The cut-tube structure 170 can be heat-set to provide a pre-defined expanded shape e.g., at the body temperature, causing the plurality of strands 173 to bend outwardly and exert a radial force against the tube member 150 of the guidewire device 100, allowing the cut-tube structure 170 to inference-fit onto the inner surface 152 of the tube member 150 of the guidewire device 100. The openings in the proximal end portion 174 and in the distal end portion 175 can be similar to or slightly greater than the cross-sectional dimension of the core wire 110 and align each other substantially on the central longitudinal axis 151 of the tube member 150, allowing the core wire 110 to be radially self-centered within the tube member 150. In alternative embodiments, the proximal end portion 174 and/or the distal end portion 175 can be secured to the core wire 110 via any suitable means such as bonding, welding, soldering, or the like. Suitable metallic shape memory materials for the tubular body structure 170 include but are not limited to alloys of nickel-titanium (NiTi) or Nitinol^{®}, CuZnAl, FeNiAl, and so on. Suitable polymeric shape-memory materials for constructing the tubular body structure 170 include but are not limited to polytetrafluoroethylene (PTFE), polylactide (PLA), ethylene-vinyl acetate (EVA), and so on.

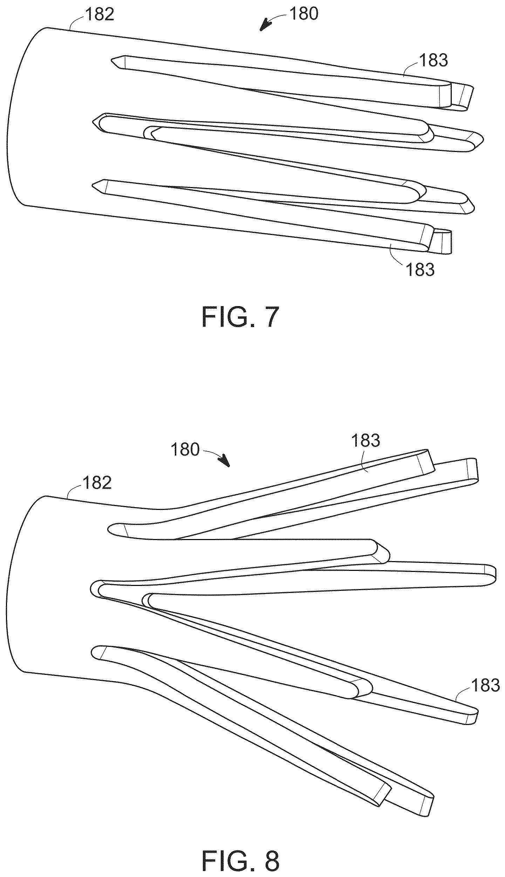

FIGS. 7-8 illustrates a further example expandable structure 180 according to alternative embodiments of the disclosure. The expandable structure 180 shown in FIGS. 7-8 comprises a spike crown-like structure 180 including an annular band portion 182 and a plurality of elongate elements 183 extending from the annular band portion 182. The spike crown-like structure 180 can have a non-expanded state (FIG. 7) and an expanded state (FIG. 8). The spike crown-like structure 180 is constructed from a shape-memory alloy or polymer and heat set to provide a pre-defined expanded shape, causing the plurality of elongate elements 183 to flex outwardly away from the annular band portion 182 and exert a radial force against the tube member 110, allowing the spike crown-like structure 180 to inference-fit onto the inner surface 152 of the tube member 150 of the guidewire device 100. The opening in the annular band portion 182 of the spike crown-like structure 180 can be similar to or slightly greater than the cross-sectional dimension of the core wire 110 and align substantially on the central longitudinal axis of the tube member 151, allowing the core wire 110 to be radially self-centered within the tube member 151. In alternative embodiments, the annular band portion 182 can be secured to the core wire 110 via a suitable means such as bonding, welding, soldering, or the like. Suitable metallic shape-memory materials for the spike crown-like structure 180 include but are not limited to alloys of nickel-titanium (NiTi) or Nitinol^{®}, CuZnAl, FeNiAl, and so on. Suitable polymeric shape-memory materials for constructing the spike crown-like structure 180 include but are not limited to polytetrafluoroethylene (PTFE), polylactide (PLA), ethylene-vinyl acetate (EVA), and so on.

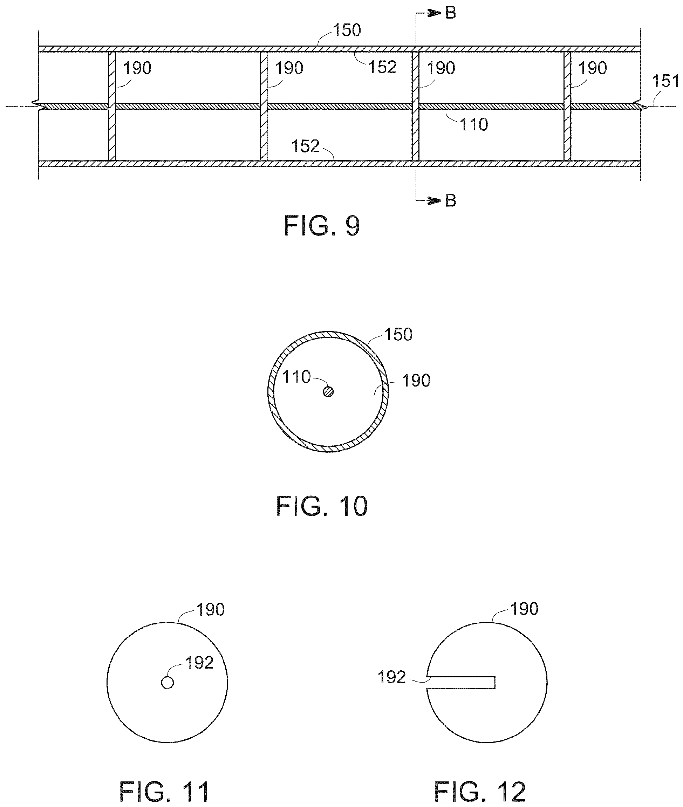

With references to FIGS. 9-12, alternative embodiments of the disclosure provide a method for centering a core wire by using disk-like structures 190. A plurality of disks 190 can be coupled to the distal portion of the elongate core wire 110. The plurality of disks 190 can be spaced apart from each other and configured to interference-fit onto an inner surface 152 of the tube member 150. Each of the plurality of disks 190 comprises an opening 192 configured to allow the core wire 110 to pass through and align substantially with the central longitudinal axis 151 of the tube member 150.

Each of the plurality of disks 190 can generally be in a circular or annular shape having a circumferential contour suitable to interference-fit onto the inner surface 152 of the tube member 150. The opening 192 in a disk 190 can be circular and centered on the central longitudinal axis 151 of the tube member 150 when the disk 190 is disposed in the tube member 150 as shown in FIG. 11. The diameter of the circular opening 192 in the disk 190 (FIG. 11) can be similar to or slightly greater than the cross-sectional diameter of the core wire 110, allowing the core wire 110 to be radially self-centered within the tube member 150. Alternatively, the disk 190 can be secured to the core wire 110 via any suitable means such as bonding, welding, soldering, or the like.

In an alternative embodiment, the opening 192 in a disk 190 can be a slot extending from a center to a periphery of the disk 190 (FIG. 12). A slot 192 in a disk 190 (Fig. 12) may ease assembly by inserting the core wire 110 into the slot 192 from the periphery of the disk 190. The confining dimension of the slot 192 can be sized similar to or slightly greater than the cross-sectional diameter of the core wire 110 to allow the core wire 110 to substantially center within the tube member 150 when in use. The disk 190 having a slot 192 (FIG. 12) can also be secured to the core wire 110 via any suitable means such as bonding, welding, soldering, or the like.

The disks 190 can be constructed from a polymer material. Suitable polymer materials include but are not limited to polyethylene, polypropylene, polyphenylene, acetal copolymer, nylon, and other suitable polymers.

The core wire centering methods of the disclosure use interference fit between the centering component(s) 160, 170, 180, 190 and the tube member 150, allowing the core wire 110 to be centered much better than the conventional centering coils because the conventional design has to have room between the centering coil and the tube member, and there is no direct contact or connection to the tube member. The centering methods of the disclosure can be easily scaled up because the expandable structure 160, 170, 180 can expand to a larger outer diameter for a tube member of a larger diameter, whereas in conventional methods redesigning of the centering coil(s) is needed as the space between the coil(s) and the tube member increases for larger guidewire devices e.g., for guidewire equal to or greater than 0.356 mm (0.014 inches). Furthermore, the expandable structure 160, 170, 180 or disks 190 can be directly secured to the core wire 110, allowing the core wire 110 and the tube member 150 in 1 to 1 alignment as the core wire 110 is rotated to improve the torque control of the guidewire device 100.

### RADIOPAQUE MAKER ON CORE WIRE DISTAL TIP

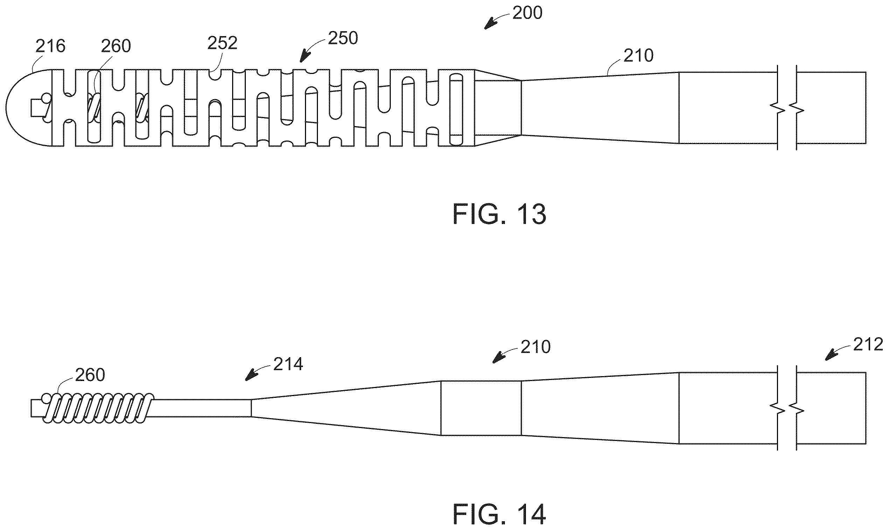

With reference to FIGS. 13 to 18, various embodiments of a guidewire device comprising a radiopaque marker on the distal end portion of a core wire are now described. The radiopaque marker comprises two or more different materials constructed to improve the shapeability and shape retention property of the guidewire device.

Shapeability and shape retention are important properties for the performance of a guidewire device. Shapeability refers to the ability of a guidewire to be manually shaped or bent at the distal end portion before use to accommodate different vascular anatomies. Having good shapeability is particularly useful for a guidewire to navigate tortuous and complex vascular pathways such as neuro or cardio vasculature. Shape retention is the ability of a guidewire to maintain the shape that has been imparted to it. It ensures that the guidewire maintains the shaped curve or angle, allowing for stable and predictable navigation.

Plastically deformable materials such as stainless steel and nickel-cobalt alloys (e.g., MP35N) have been used to construct core wires due to their shapeability and shape retention properties. A plastically deformable material can undergo deformation when subjected to stress beyond its elastic limit without fracturing. This ability, or plasticity, allows the material to change shape and maintain the new form after the applied force is removed. On the other hand, shape-memory hypotubes have been used as components in guidewire devices to improve the performance. For instance, some conventional guidewire devices include a shape-memory slotted hypotube at the distal tip of a core wire to improve the torque control of the device. A radiopaque coil of a pure metal such as platinum (Pt) is typically disposed within the hypotube for visualization of the location of the distal tip of the core wire. While a hypotube on a guidewire distal tip can improve the torqueability of the device, its shape-memory property would compel it to return to its original or pre-defined configuration after deformation when exposed to the body temperature. As such, a shape-memory hypotube may adversely impact the shapeability and shape retention properties of the guidewire device due to its tendency of returning to the original or pre-defined configuration.

According to embodiments of the disclosure, a radiopaque marker constructed of two or more different materials is used to help maintain or improve the shapeability and shape retention properties of a guidewire device. As shown in FIG. 13, an example guidewire device 200 of the disclosure comprises an elongate core wire 210 extending between a proximal portion 212 and a distal portion 214, a tube member 250 coupled to the distal portion 214 of the core wire 210, and a radiopaque marker 260 in the tube member 250 and coupled to the core wire 210. The radiopaque marker 260 comprises a radiopaque first material and a plastically deformable second material. While the radiopaque first material provides radiopacity, the plastically deformable second material helps maintain the shape that has been imparted to the distal portion of the guidewire device 200. In an embodiment, the tube member 250 is constructed from a shape-memory material and comprises a plurality of cuts 252 circumferentially extending around a central longitudinal axis of the tube member. The radiopaque marker 260 of the disclosure can provide resistance against the tendency of the shape-memory tube member 250 of returning to its original or pre-defined configuration, thus improving the shapeability and retention properties of the guidewire device 200.

According to embodiments of the disclosure, the radiopaque first material of the radiopaque marker can be any suitable radiopaque material visible via fluoroscopy, including but not limited to tungsten, platinum, iridium, gold, tantalum, or any alloy thereof such as a platinum-iridium alloy, a platinum-tungsten alloy, and so on.

According to embodiments of the disclosure, the plastically deformable second material of the radiopaque marker can be a metal, a metal alloy, a metal composite, including but not limited to stainless steel, nickel-cobalt (e.g., MP35N), a nickel-titanium alloy (e.g., Nitinol), a cobalt-chromium alloy, a platinum alloy, a titanium alloy, and so on.

With reference to FIG. 14, according to embodiments of the disclosure the radiopaque marker 260 can be in the form of a coil wound around a section of the core wire distal end portion 214. The radiopaque coil 260 can be secured to the core wire 210 via bonding, welding, soldering, crimping, or any other suitable means. The pitch of the radiopaque coil 260 can be constant or vary. By way of example, the radiopaque coil 260 may have a varying winding pitch increasing toward the distal end to improve the flexibility of the guidewire tip, and/or reduce the stiffness caused by the plastically deformable material used in the radiopaque coil 260.

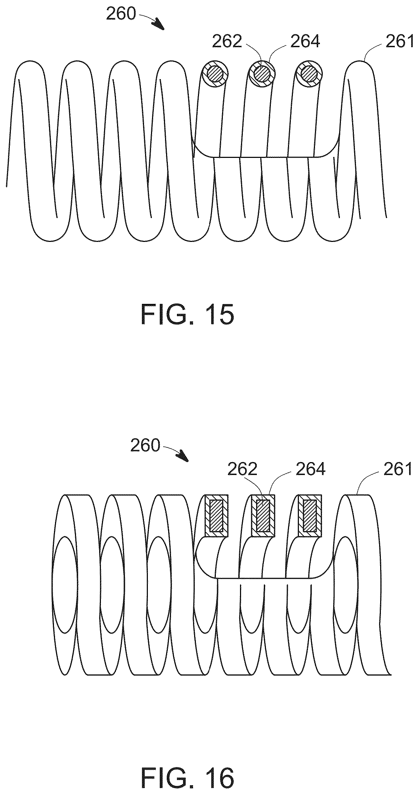

With reference to FIGS. 15-16, according to embodiments of the disclosure the radiopaque marker coil 260 can be formed of a metal composite wire 261 comprising an inner core 262 and outer sheath 264. For example, the metal composite wire 261 can be a drawn-filled tubing (DFT) wire comprising an inner core 262 of a radiopaque material such as tungsten, platinum, iridium, gold, tantalum, or any alloy thereof, and an outer sheath 264 of a plastically deformable material such as stainless steel, a nickel-cobalt alloy, a nickel-titanium alloy, a cobalt-chromium alloy, a platinum alloy, a titanium alloy, etc. In some embodiments, the metal composite wire 261 can be a round wire or have a circular or substantially circular cross-section, as shown in FIG. 15. In some embodiments, the metal composite wire 261 can be a ribbon or flat wire or have a cross-section in a non-circular shape such as a rectangular shape shown in FIG. 16 or other shapes where the inner core cross-sectional area comprises a wider dimension and a narrower dimension. Using a ribbon or flat metal composite wire 261 (FIG. 16), the radiopaque marker coil 260 can be formed such that when being secured to the distal portion 214 of the core wire 210, the wider dimension of the inner core 262 is in the radial direction. This arrangement or design would allow more radiopaque material to block radiation during fluoroscopy, thereby increasing the radiopacity of the marker coil 260.

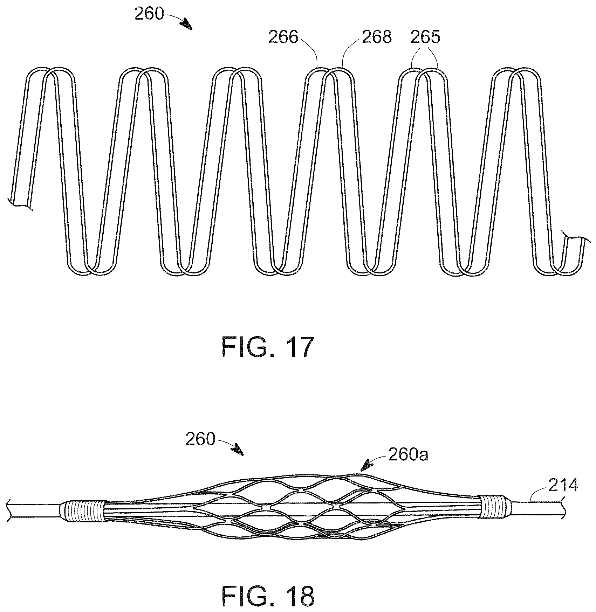

With reference to FIG. 17, according to embodiments of the disclosure the radiopaque marker coil 260 can be formed of a bifilar wire 265 comprising a first wire 266 and a second wire 268 parallel with the first wire 266. The first wire 266 of the bifilar wire 265 may comprise a radiopaque material such as tungsten, platinum, iridium, gold, tantalum, or any alloy thereof. The second wire 268 of the bifilar wire 265 may comprise a plastically deformable material such as stainless steel, a nickel-cobalt alloy, a nickel-titanium alloy, a cobalt-chromium alloy, a platinum alloy, a titanium alloy, etc. The first wire 266 of the radiopaque material and the second wire 268 of plastically deformable material can be co-wound around a section of the core wire distal portion 214, allowing the first wire 266 of the radiopaque material and the second wire 268 of plastically deformable material to alternate but not to cross one another in the radiopaque marker coil 260.

With reference to FIG. 18, according to embodiments of the disclosure the radiopaque marker 260 can be in the form of a braid. A radiopaque braid 260a would provide greater shape retention capabilities than a radiopaque coil because there is friction between each crossing point of the braid. A radiopaque braid 260a may be thicker than a radiopaque coil and can be more useful in guidewire devices of larger sizes such as 0.610 mm (0.024 inches) or 0.889 mm (0.035 inches). The radiopaque braid 260a can be secured to the core wire distal portion 214 via crimping, bonding, welding, soldering, or any other suitable means.

The radiopaque braid 260a can be constructed from two or more metal composite wires such as DFT wires. Each of the two or more DFT wires can comprise an inner core of a radiopaque material such as tungsten, platinum, iridium, gold, tantalum, or any alloy thereof, and an outer sheath of a plastically deformable material such as stainless steel, a nickel-cobalt alloy, a nickel-titanium alloy, a cobalt-chromium alloy, a platinum alloy, a titanium alloy, etc. as discussed above in conjunction with a radiopaque coil.

According to embodiments of the disclosure, the radiopaque braid 260a can be formed of two or more different wires. The two or more different wires may comprise a first wire of a radiopaque material such as tungsten, platinum, iridium, gold, tantalum, or any alloy thereof, and a second wire of a plastically deformable material such as stainless steel, a nickel-cobalt alloy, a nickel-titanium alloy, a cobalt-chromium alloy, a platinum alloy, a titanium alloy, etc.

Returning to FIG. 13, according to embodiments of the disclosure the guidewire device 200 may comprise a radiopaque tip 216 coupled to the distal end of the core wire 210 and/or tube member 250. The radiopaque tip 216 can be a radiopaque metallic ball such as platinum or gold ball secured to the core wire tip via bonding, soldering, welding, or other suitable means. Alternatively, a radiopaque solder or epoxy can be used to form a radiopaque tip 216 at the distal end of the core wire 210 and/or tube member 250. While a radiopaque coil 260 or braid 260a comprising a plastically deformable material might reduce the radiopacity of the marker coil or braid, the use of a gold solder, gold epoxy, platinum ball or other radiopaque materials in the tip 216 would allow the physician to easily identify the dark spot representing the radiopaque tip of the guidewire device 200.

Advantageously, embodiments of the disclosure include a plastically deformable material in a radiopaque marker to improve the shapeability and shape retention properties of a guidewire device. Conventional guidewire devices, especially shape-memory hypotube-based guidewire devices, use a pure metal coil such as platinum and tantalum coil on the distal end of the core wire for radiopacity and have issues with it being difficult to shape the guidewire tip or retain the shape imparted onto it when in use. The radiopaque marker of the disclosure can be constructed from a metal composite wire comprising an outer sheath of plastically deformable material, or constructed from a bifilar wire comprising a wire of a plastically deformable material, or in the form of a braid where at least one of the wires making the braid includes a plastically deformable material. The radiopaque marker of the disclosure comprising a plastically deformable material significantly improves the shapeability and shape retention properties of the guidewire device. The pitch of the marker coil can be varied across its length to account for stiffness of the plastically deformable material. The guidewire atraumatic tip can be constructed of a radiopaque material to account for lighter coloration due to the use of a plastically deformable material. Alternatively, or in addition, a ribbon or flat metal composite wire can be used to increase radiopacity of the marker coil.

### CORE WIRE SUPPORT RAMP-UP INDICATOR

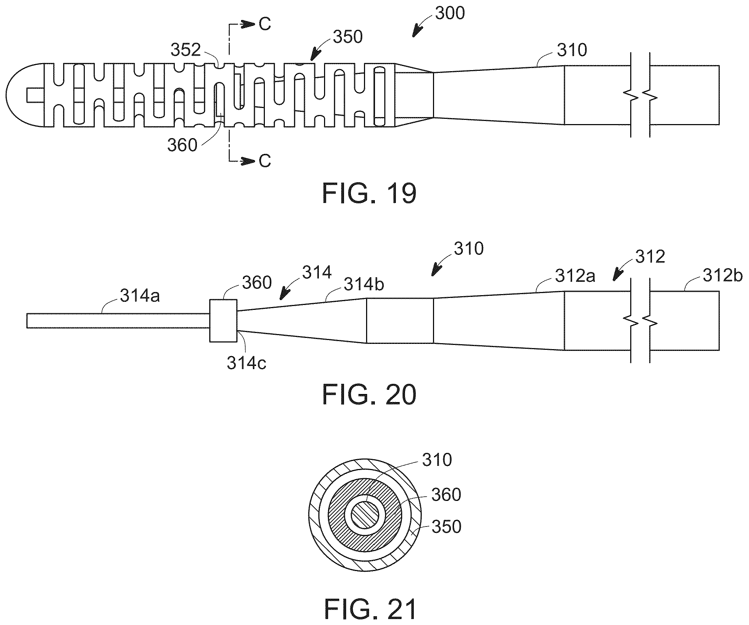

With reference to FIGS. 19 to 21, embodiments of a guidewire device comprising an indicator for indicating change of the support or stiffness profile of a core wire are now described.

Guidewires are often offered by manufacturers with a variety of support or stiffness profiles e.g., a soft profile, a standard profile, and a support profile, etc. Different support or stiffness profiles provide the physician with options when it comes to access and delivery of ancillary products. For example, a guidewire with a softer profile may be preferred when accessing more distal anatomy whereas a stiffer guide wire may be considered for delivery of heavier devices, such as balloons.

A guidewire device includes a core wire typically running the length of the device. The core wire serves as central structural support providing a stiffness profile for pushability, torque transmission, and flexibility for the guidewire device to navigate vascular pathways. To achieve a desired stiffness or support profile, the shape or contour of the core wire can be varied along the length e.g. by grinding. Grinding away more material results in a softer core wire or a softer section of the core wire, and vice versa. There is often a notable point in the grind profile where the stiffness ramps up more drastically than in other locations, and this increase in stiffness can be felt on the assembled guidewire and directly impact the performance of the device. It would be desirable to provide a clear visual indication where the change of support profile such as a stiffness ramp-up occurs. Knowing where the support profile ramps up during use can help the physician position the guidewire appropriately during delivery of ancillary products.

With reference to FIGS. 19-21, an example guidewire device 300 according to embodiments of the disclosure comprises an elongate core wire 310 having a varying support or stiffness profile and an indicator 360 secured to the core wire 310 to provide a visual indication of a change of the stiffness profile of the core wire 310. The guidewire device 300 may also include a tubular sleeve 350 or a polymer jacket near or over the distal portion of the core wire 310 to provide reinforcement. As shown, an example tubular sleeve or tube member 350 may comprise a plurality of cuts or slots 352 configured to improve the effectiveness of the guidewire device. While not shown in FIGS. 19-21, the guidewire device 300 may also include other components such as a component for radially centering the core wire in the tube member and/or a distal radiopaque marker as described in conjunction with other embodiments of the disclosure, or other components known in the art.

With reference to FIG. 20, the elongate core wire 310 may extend between a proximal portion 312 and a distal portion 314 e.g., running the length of the guidewire device 300. The elongate core wire 310 may be constructed from a single continuous piece of a material such as stainless steel, a cobalt-chromium alloy, a nickel-titanium alloy, a platinum alloy, a titanium alloy, or a DFT composite as will be described in greater detail hereafter. The core wire 310 may also be constructed from two or more segments of different materials joined e.g., via welding, soldering, bonding, or mechanical interlocking etc.

With reference to FIG. 20, the core wire 310 has a varying profile or geometry along the length of the core wire 310. In general, the distal portion 314 of the core wire 310 has a reduced profile to optimize flexibility of the guidewire device 300 at the distal end for enhanced maneuverability. The proximal portion 312 of the core wire 310 may have an enlarged profile to maintain pushability and torsional rigidity of the guidewire device 300. The core wire 310 may include one or more tapered sections in the distal portion 314 and proximal portion 312 to facilitate gradual transition from a reduced profile to an enlarged profile.

By way of example, the distal portion 314 of the core wire 310 may include a distal or first section 314a and a proximal or second section 314b. The first section 314a of the distal portion 314 may have a profile or geometry having a constant cross-sectional dimension. For example, the first section 314a of the distal portion 314 may be flattened or rounded having a constant cross-sectional dimension e.g., in a constant rectangular, circular, oval shape or the like. The second section 314b of the distal portion 314 may be tapered having varying cross-sectional dimensions e.g., diameters or areas increasing in the proximal direction.

Likewise, the proximal portion 312 of the core wire 310 may include a distal or first section 312a and a proximal or second section 312b. The second section 312b of the proximal portion 312 may have a profile or geometry having a constant cross-sectional dimension. For example, the second section 312b of the proximal portion 312 may be cylindrical having a constant diameter. The first section 312a of the proximal portion 312 may be tapered having varying cross-sectional dimensions e.g., diameters or areas decreasing in the distal direction.

With reference to FIG. 20, the tapered section 314b at the distal portion 314 and/or the tapered section 312a at the proximal portion 312 of the core wire 310 may have a taper angle with respect to the longitudinal axis of the core wire, ranging from 0.01 degrees to 0.06 degrees. The value of a taper angle indicates the drasticness of change of the stiffness profile of the core wire 310. A smaller taper angle indicates a gradual or incremental change of the stiffness profile whereas a greater taper angle indicates a drastic ramp-up of stiffness profile. By way of example, the tapered section 314b at the distal portion 314 of the core wire 314 may have a taper angle ranging from 0.02 degrees to 0.05 degrees.

According to embodiments of the disclosure, an indicator 360 is provided in the distal portion 314 near the tapered section 314b, e.g., at the joint 314c between the constant first section 314a and the tapered second section 314b. The indicator 360 is configured to provide a visual indication of change of stiffness profile of the core wire 310.

The indicator 360 can be a radiopaque indicator visible via fluoroscopy or computed tomography (CT). Alternatively, or in addition, the indicator 360 can be other indicators visible via other imaging modalities such as ultrasound, magnetic resonance imaging (MRI), etc. Suitable materials for a radiopaque indicator 360 include heavy metals or metal alloys, including but not limited to tungsten, platinum, iridium, gold, tantalum, or any alloy thereof such as a platinum-iridium alloy, a platinum-tungsten alloy, and so on. The radiopaque indicator 360 can be in the form of a marker band or coil or any other suitable forms.

The indicator 360 can be secured to the core wire 310 through a variety of means, such as crimping and/or laser welding. Alternatively, or in addition, the indicator 360 can be secured to the tube member 350 through the use of glue and/or solder, which can sink into the cuts or slots 352 in the tube member 350. A combination of securement methods can also be used. For example, a laser weld, crimp, glue, or solder can be used to lightly secure the indicator 360 to the core wire 310 such that further assembly with the tube member 350 does not shift the indicator 360 before it is secured to the tube member 350 with glue, solder, laser welding, etc.

The indicator 360 according to embodiments of the disclosure provides the physician with a visual indication where a support profile ramp-up of the guidewire device 300 occurs during clinical use. Knowing where the support profile ramps up during use can help the physician position the guidewire device 300 appropriately during delivery of ancillary products. Another benefit is that the indicator 360 can help center the core wire 310 within the slotted tube member 350. Radially centering of the core wire 310 within the tube member 350 help provide more uniform torque transmission. A further benefit is that the indicator 360 can help prevent the slotted tube member 350 from stretching, which otherwise would adversely affect pushability of the guidewire device 300 during use. The indicator 360 can be secured to both the tube member 350 and the core wire 310, providing friction between the tube member 350 and the core wire 310 and thus prevents the tube member 350 from stretching.

### METAL COMPOSITE CORE WIRE

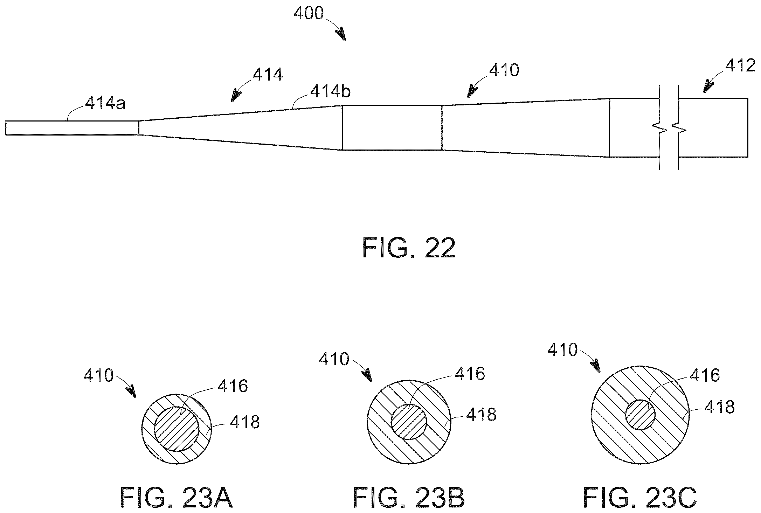

With reference to FIGS. 22 and 23, embodiments of a core wire for use in a guidewire and other medical devices are now described.

A guidewire device includes a core wire typically running the length of the guidewire. The core wire material is generally selected to balance the need for torque response, tip shapeability, tip softness, support or stiffness profile, and so on. One issue in the art is that there are limited core wire materials to choose from and it is impossible to choose an intermediate. While stainless steel, cobalt-chromium alloys, and nitinol have been used to construct core wires, each of the materials has advantages and disadvantages. For instance, a core wire constructed from nitinol can provide super tip softness but less desirable support profile and torque response. A core wire constructed from a cobalt-chromium alloy can provide good support profile and torque response but poor tip shapeability. To achieve a desired set of properties for a guidewire device, the design has to be compensated with the other components such as an exterior jacket or tubular sleeve.

Another issue in the art especially associated with small guidewire devices (e.g., 0.254 mm (0.010 inches) or smaller) is radiopacity. Conventional core wire materials are non-radiopaque. Therefore, the design of a conventional guidewire has to include a radiopaque marker of a dense material such as platinum to provide an appropriate level of visibility under fluoroscopy. If a polymer jacket is used as a guidewire component, it is possible to load a radiopaque material such as tungsten or barium sulfate to the polymer to improve radiopacity. However, if a polymer jacket is not in the design, then the only source of radiopacity would be a radiopaque coil on the tip of the core wire. For smaller guidewire devices, there is limited space for a radiopaque coil and increasing its size will often reduce other benefits.

According to embodiments of the disclosure, a metal composite wire is used to construct a core wire, which overcomes these and other problems associated with the conventional guidewires.

FIG. 22 schematically shows an example guidewire device comprising a core wire according to embodiments of the disclosure. FIGS. 23A, 23B, and 23C are simplified illustrations showing cross-sectional end views of example core wires. As shown in FIGS. 22 and 23A-23C, the example guidewire device 400 comprises an elongate core wire 410 extending between a proximal portion 412 and a distal portion 414. The core wire 410 can be formed of a metal composite wire comprising an inner core 416 and an outer sheath 418 both extending the length of the core wire 410. The inner core 416 of the metal composite wire 410 comprises a first material. The outer sheath 418 of the metal composite wire 410 comprises a second material different from the first material of the inner core 416. While not shown in FIGS. 22 and 23A-23C, the guidewire device 400 may also include a polymer jacket or a tubular or coil sleeve disposed over the distal portion 414 of the core wire 410, a component for centering the core wire 410 in the tube sleeve and/or a distal radiopaque marker as described in conjunction with other embodiments of the disclosure, or other components known in the art.

According to embodiments of the disclosure, the first material of the metal composite inner core 416 comprises a radiopaque material. One advantage of using a radiopaque material as the metal composite inner core 416 is that it allows the core wire 410 itself to be visible via fluoroscopy. This is particularly beneficial for small diameter guidewires (e.g., 0.254 mm (0.010 inches) or smaller) using a tubular sleeve as reinforcement which provides limited space for a radiopaque coil. For larger guidewires provided with a radiopaque coil at the distal tip, a metal composite core wire including an elongate radiopaque inner core 416 can also improve the overall radiopacity of the guidewire device. Suitable radiopaque materials that can be used as the metal composite inner core 416 include but are not limited to gold, silver, platinum, tantalum, tungsten, and any alloys thereof.

In some embodiment, the metal composite inner core 416 comprises a non-radiopaque material e.g., nitinol to take advantage of e.g., the softness property of nitinol. Alternatively, the metal composite inner core comprises a cobalt-chromium alloy, a platinum alloy, a titanium alloy, or stainless steel.

According to embodiments of the disclosure, the second material of the metal composite outer sheath 418 comprises a material different from the first material of the inner core 416 to provide other desirable properties such as strength, flexibility, elasticity, etc. Suitable materials for the metal composite outer sheath 418 include but are not limited a cobalt-chromium alloy, a nickel-titanium alloy, a platinum alloy, or a titanium alloy, stainless steel, etc. Stainless steel can provide the core wire with relatively balanced performance in terms of torque response, support profile, tip shapeability, and tip softness. A cobalt-chromium alloy can provide better performance in torque response and support profile, although the tip shapeability and tip softness are less desirable. A nickel-titanium alloy (nitinol) can provide superb tip softness but less desirable tip shapeability, torque response, and support profile.

Table 1 provides example materials which can be used for the outer sheath of a metal composite wire to construct the core wire 410 of the disclosure, with their advantages or disadvantages being compared.

**Table 1**

| | Stainless Steel | Cobalt-Chromium | Nickel-Titanium | Cobalt-Chromium /Platinum Composite |
|---|---|---|---|---|
| Torque Response | +++ | ++++ | ++ | ++++ |
| Support Profile | +++ | ++++ | ++ | ++++ |
| Tip shapeability | +++ | + | + | ++ |
| Tip softness | +++ | ++ | +++++ | +++ |
| Tip Support Profile | +++ | ++++ | ++ | +++ |

Therefore, according to embodiments of the disclosure, the materials of both the inner core 416 and the outer sheath 418 of a metal composite core wire 410 can be varied to provide a variety of performance benefits. In conventional guidewire manufacturing, there is typically no middle ground in selecting core wire materials. The metal composite core wire 410 of the disclosure provide intermediates. By way of example, as shown in Table 1, using a metal composite core wire 410 with a cobalt-chromium outer sheath 418 and a platinum inner core 416 fill would allow greater shapeability due to the increased ductility of the platinum, and improve the tip softness due to platinum being softer than cobalt-chromium. While there may be a tradeoff associated with the tip support profile, the support profile of the cobalt-chromium/platinum composite core wire 410 is no worse than stainless steel.

According to an embodiment, a metal composite core wire 410 of the disclosure comprises an inner core 416 of a radiopaque material selected from the group consisting of gold, silver, platinum, tantalum, tungsten, and any alloy thereof.

According to an embodiment, a metal composite core wire 410 of the disclosure comprises an outer sheath 418 of a material selected from the group consisting of a cobalt-chromium alloy, a nickel-titanium alloy, a platinum alloys, a titanium alloy, and stainless steel.

According to an embodiment, a metal composite core wire 410 of the disclosure comprises an inner core 416 of a radiopaque material selected from the group consisting of gold, silver, platinum, tantalum, tungsten, and an alloy thereof, and an outer sheath 418 of a material selected from the group consisting of a cobalt-chromium alloy, a nickel-titanium alloy, a platinum alloys, a titanium alloy, and stainless steel.

According to a specific embodiment, a metal composite core wire 410 of the disclosure comprises an inner core 416 of platinum and an outer sheath 418 of a cobalt-chromium alloy.

According to a specific embodiment, the metal composite core wire 410 of the disclosure comprises a drawn-filled tubing (DFT) wire comprising an inner core 416 and an outer sheath 418.

With reference to FIGS. 23A-23C, according to embodiments of the disclosure the fill percentage of the metal composite core wire 410 can also be adjusted to preferentially focus on the benefits of one material over the other. As used herein, the phrase "fill percentage" refers to the proportion of the metal composite wire's cross-sectional area occupied by the inner core 416 material (filled material) relative to the total cross-sectional area of the metal composite wire 410. According to embodiments of the disclosure, the metal composite core wire 410 comprises a fill percentage of the inner core 416 material ranging from about 10 to 45 percent along the length of the core wire 410.

According to embodiments of the disclosure, the fill percentage of the metal composite core wire 410 may vary along the length of the core wire 410. By way of example, the metal composite core wire 410 may comprise a first fill percentage at the proximal portion 412 of the core wire 410 and a second fill percentage at the distal portion 414 of the core wire 410 different from e.g., greater than the first fill percentage. This would allow the material properties of the inner core 416 to be more dominant at the distal portion 414 of the core wire 410 and the material properties of the outer sheath 418 more dominant at the proximal portion 412 of the core wire 410. For example, a metal composite core wire 410 of the disclosure may comprise an inner core 416 of platinum and an outer sheath 418 of a cobalt-chromium alloy. A greater fill percentage of the platinum inner core 416 at the distal portion 414 of the metal composite core wire 410 as shown in FIG. 23A would be beneficial in improving the softness, shapeability, and radiopacity of the core wire 410. Alternatively, or in addition, a smaller fill percentage of the platinum inner core 416 at the proximal portion 412 of the metal composite core wire 410 as shown in FIG. 23C would focus more on the support profile and torque response of the core wire 410.

According to embodiments of the disclosure, the metal composite core wire 410 may comprise a constant fill percentage at a portion of the metal composite core wire 410. Alternatively, or in addition, the metal composite core wire 410 may comprise a varying or continuously varying fill percentage at a portion of the metal composite core wire 410. By way of example, in the proximal portion 412 of the core wire 410, the metal composite core wire 410 may have a generally constant small fill percentage of the inner core 416 material. In the distal portion 414 of the core wire 410, which may include one or more constant sections and one or more tapered sections, the fill percentage of the metal composite core wire can be varied. For example, the distal portion 414 of the metal composite core wire 410 may comprise a distal first section 414a having a constant diameter or cross-section and a proximal tapered or second section 414b having a varying diameter or cross-section. The fill percentage of the metal composite core wire 410 at the constant first section 414a may be constant whereas the fill percentage of the metal composite core wire 410 at the tapered second section 414b may be continuously varied e.g., decreasing in the proximal direction.

According to embodiments of the disclosure, a raw metal composite wire 410 can be ground to remove a portion of the outer sheath 418 material to achieve a desirable fill percentage of the metal composite core wire 410. For example, a greater fill percentage can be achieved by grinding more outer sheath 418 materials at the distal portion 414 of the core wire 410 as shown in FIG. 23A, whereas a smaller fill percentage can be achieved by grinding less outer sheath 418 materials at the proximal portion 412 of the core wire 410 as shown in FIG. 23C. Alternatively, or in addition, a varying fill percentage can be achieved by gradually grinding the metal composite wire 410 to form a tapered segment.

According to embodiments of the disclosure, the metal composite core wire 410 of the disclosure may have an outer diameter ranging from 0.018 mm to 3.175 mm (0.0007 inches to 0.125 inches) at the proximal portion 412. In some embodiments, the metal composite core wire has an outer diameter ranging from 0.178 mm to 0.889 mm (0.007 inches to 0.035 inches) at the proximal portion 412.

Various embodiments of guidewire devices and methods have been described with reference to figures. It should be noted that an aspect described in conjunction with a particular embodiment is not necessarily limited to that embodiment and can be practiced in any other embodiments. For example, the embodiments of centering core wires described in conjunction with FIGS. 1-12 can be incorporated in the embodiments of radiopaque coil or braid described in conjunction with FIGS. 13-18 or vice versa, can be incorporated in the embodiments of core wire support indicators described in conjunction with FIGS. 19-21 or vice versa, and can be incorporated in the embodiments of metal composite core wires described in conjunction with FIGS. 22 and 23A-23C or vice versa. Likewise, the embodiments of radiopaque coil or braid described in conjunction with FIGS. 13-18, the embodiments of core wire support indicators described in conjunction with FIGS. 19-21, and the embodiments of metal composite cores wire described in conjunction with FIGS. 22 and 23A-23C, can each be incorporated in any other embodiments or aspects of the disclosure described herein.

The figures are intended for illustration of embodiments but not for exhaustive description or limitation on the scope of the disclosure. Alternative structures, components, and materials will be readily recognized as being viable without departing from the principle of the claimed invention. Further, while some embodiments of the disclosure are described in conjunction with a guidewire device, this is not intended to be limiting. For example, the core wires described herein can be used as a component for other interventional devices.

All technical and scientific terms used herein have the meaning as commonly understood by one of ordinary skill in the art unless specifically defined otherwise. As used in the description and appended claims, the singular forms of "a," "an," and "the" include plural references unless the context clearly dictates otherwise. The term "or" refers to a nonexclusive "or" unless the context clearly dictates otherwise. The term "proximal" and its grammatically equivalent refers to a position, direction or orientation towards the user or physician's side. The term "distal" and its grammatically equivalent refers to a position, direction, or orientation away from the user or physician's side. The designations "rearward," "forward," and the like are not meant to limit the referenced component to a specific orientation. It will be appreciated that such designations refer to the orientation of the referenced component as illustrated in the Figures; the systems and devices of the disclosure can be used in any orientation suitable to the user. The term "first" or "second" etc. may be used to distinguish one element from another in describing various similar elements. It should be noted the terms "first" and "second" as used herein include references to two or more than two. Further, the use of the term "first" or "second" should not be construed as in any particular order unless the context clearly dictates otherwise. The order in which the method steps are performed may be changed in alternative embodiments. One or more method steps may be skipped altogether, and one or more optional steps may be included. All numeric values are provided for illustration and assumed to be modified by the term "about," whether explicitly indicated or not. The term "about" generally refers to a range of numbers that one of skill in the art would consider equivalent to the recited value e.g., having the same function or result. The term "about" may include numbers that are rounded to the nearest significant figure. The recitation of a numerical range by endpoints includes all numbers within that range.

Particular aspects of the subject-matter disclosed herein are set out in the following numbered clauses. The embodiments disclosed in the following clauses may be pursued in the present application or in any divisional application.
1. A guidewire device comprising: a core wire extending between a proximal portion and a distal portion; a tube member located near the distal portion of the core wire, the tube member being secured to the core wire and defining a space between the core wire and the tube member; and an expandable structure disposed in the space between the core wire and the tube member, the expandable structure being configured to interference-fit onto an inner surface of the tube member and comprising a proximal end having an opening and a distal end having an opening to allow the core wire to pass through the expandable structure, wherein the opening of the proximal end and the opening of the distal end of the expandable structure substantially align with a central longitudinal axis of the tube member and are configured to encircle the core wire to thereby align the core wire substantially with the central longitudinal axis of the tube member.
2. The guidewire device of clause 1, wherein the expandable structure comprises a stent-like structure.
3. The guidewire device of clause 2, wherein the stent-like structure comprises a braid constructed from shape-memory filaments.
4. The guidewire device of clause 2, wherein the proximal end and/or the distal end of the stent-like structure are/is fixedly secured to the core wire.
5. The guidewire device of clause 1, wherein the expandable structure comprises a cut-tube structure, the cut-tube structure comprising a tubular body constructed from a shape-memory material and provided with a plurality of longitudinal slits to form a plurality of strands extending between a proximal end portion and a distal end portion of the tubular body, wherein the cut-tube structure is heat set to provide an expanded shape where the plurality of strands bend outwardly and exert a radial force against the tube member, thereby allowing cut-tube structure to inference-fit onto the inner surface of the tube member of the guidewire device.
6. The guidewire device of clause 5, wherein the tubular body is constructed from a nickel-titanium alloy.
7. The guidewire device of clause 5, wherein the proximal end portion and the distal end portion of the tubular body are fixedly secured to the core wire.
8. The guidewire device of clause 1, wherein the expandable structure comprises a spike crown-like structure comprising an annular band portion and a plurality of elongate elements extending from the annular band portion, wherein the spike crown-like structure is constructed from a shape-memory material and heat set to provide an expanded shape where the plurality of elongate elements flex outwardly away from the annular band portion and exert a radial force against the tube member, thereby allowing the spike crown-like structure to inference-fit onto the inner surface of the tube member of the guidewire device.
9. The guidewire device of clause 8, wherein the spike crown-like structure is constructed from a nickel-titanium alloy.
10. The guidewire device of clause 8, wherein the annular band portion of the spike crown-like structure is fixedly secured to the core wire.
11. The guidewire device of clause 1, wherein the proximal portion of the core wire has an outer diameter equal to or greater than 0.014 inches.
12. The guidewire device of clause 1, wherein the proximal portion of the core wire has an outer diameter ranging between 0.014 inches and 0.038 inches.
13. The guidewire device of clause 1, wherein the tube member comprises a plurality of cuts circumferentially extending around the central longitudinal axis of the tube member.
14. A guidewire device comprising: a core wire extending between a proximal portion and a distal portion; a tube member located near the distal portion of the core wire, the tube member being secured to the core wire and defining a space between the core wire and the tube member; and a plurality of disks coupled to the distal portion of the core wire, the plurality of disks being spaced apart from each other and configured to interference-fit onto an inner surface of the tube member, wherein the plurality of disks each comprises an opening configured to allow the core wire to pass through and align substantially with a central longitudinal axis of the tube member.
15. The guidewire device of clause 14, wherein the plurality of disks is constructed from a polymeric material.
16. The guidewire device of clause 14, wherein the opening of one or more of the plurality of disks is circular.
17. The guidewire device of clause 14, wherein the opening of one or more of the plurality of disks is a slot extending from a center to a periphery of the one or more of the plurality of disks.
18. The guidewire device of clause 14, wherein the tube member comprises a plurality of cuts circumferentially extending around the central longitudinal axis of the tube member.
19. The guidewire device of clause 18, wherein the plurality of disks is fixedly secured to the core wire.
20. The guidewire device of clause 19, wherein the plurality of disks is further fixedly secured to the tube member.
21. The guidewire device of clause 14, wherein the proximal portion of the core wire has an outer diameter equal to or greater than 0.014 inches.
22. The guidewire device of clause 14, wherein the proximal portion of the core wire has an outer diameter in the range between 0.014 inches and 0.038 inches.
23. A guidewire device comprising: a core wire extending between a proximal portion and a distal portion; a tube member located near the distal portion of the core wire and coupled to the core wire; and a radiopaque marker in the tube member and coupled to the core wire, wherein the radiopaque marker comprises a radiopaque first material and a plastically deformable second material.
24. The guidewire device of clause 23, wherein the radiopaque marker is in the form of a coil wound around the distal portion of the core wire.
25. The guidewire device of clause 24, wherein the coil is formed of a metal composite wire comprising an inner core of the radiopaque first material and an outer sheath of the plastically deformable second material.
26. The guidewire device of clause 25, wherein the metal composite wire has a cross-section of the inner core in a non-circular shape comprising a wider dimension and a narrower dimension, and wherein the metal composite wire is wound around the distal portion of the core wire such that the wider dimension of the non-circular shape is in the radial direction to improve radiopacity of the radiopaque marker.
27. The guidewire device of clause 25, wherein the coil comprises a varying pitch increasing in a distal direction to improve softness of the distal portion of the core wire.
28. The guidewire device of clause 25, wherein the plastically deformable second material of the outer sheath of the metal composite wire comprises a nickel-cobalt alloy or stainless steel.
29. The guidewire device of clause 24, wherein the coil comprises a bifilar wire comprising a first wire and a second wire parallel with the first wire, the first wire of the bifilar wire comprising the radiopaque first material, and the second wire of the bifilar wire comprising the plastically deformable second material.
30. The guidewire device of clause 29, wherein the plastically deformable second material comprises a nickel-cobalt alloy or stainless steel.
31. The guidewire device of clause 29, wherein the bifilar wire winds around the distal portion of the core wire with a varying pitch increasing in a distal direction to improve softness of the distal portion of the core wire.
32. The guidewire device of clause 23, wherein the radiopaque marker is in the form of a braid.
33. The guidewire device of clause 32, wherein the braid is constructed from two or more metal composite wires, each of the two or more metal composite wires comprising an inner core of the radiopaque first material and an outer sheath of the plastically deformable second material.
34. The guidewire device of clause 32, wherein the braid is constructed from two or more wires comprising a first wire of the radiopaque first material and a second wire of the plastically deformable second material.
35. The guidewire device of clause 32, wherein the core wire has an outer diameter equal to or greater than 0.024 inches at the proximal portion of the core wire.
36. The guidewire device of clause 23, further comprising a rounded tip coupled to a distal end of the core wire, wherein the rounded tip comprises a radiopaque material.
37. The guidewire device of clause 23, wherein the tube member is constructed from a shape-memory alloy and comprises a plurality of cuts circumferentially extending around a central longitudinal axis of the tube member.
38. A guidewire device comprising: a core wire extending between a proximal portion and a distal portion, the distal portion comprising a first section having a first stiffness profile and a second section having a second stiffness profile different from the first stiffness profile; and an indicator secured to the core wire, wherein the indicator is located at a joint of the first section and the second section to provide a visual indication of a change of stiffness profile of the core wire.
39. The guidewire device of clause 38, wherein the first section has a substantially constant cross-sectional dimension.
40. The guidewire device of clause 39, wherein the second section is tapered with cross-sectional dimensions increasing in a proximal direction.
41. The guidewire device of clause 40, wherein the second section has a taper angle equal to or greater than 0.02 degrees.
42. The guidewire device of clause 38, wherein the indicator comprises a radiopaque indicator.
43. The guidewire device of clause 42, wherein the radiopaque indicator is in the form of a marker band or coil.
44. The guidewire device of clause 38, further comprising a tube member located near the distal portion of the core wire, the tube member comprising a plurality of cuts circumferentially extending around a central longitudinal axis of the tube member.
45. The guidewire device of clause 44, wherein the indicator is further secured to the tube member.
46. A guidewire device comprising a core wire extending between a proximal portion and a distal portion, wherein the core wire comprises a metal composite wire comprising an inner core of a first material and an outer sheath of a second material different from the first material.
47. The guidewire device of clause 46, wherein the first material of the inner core comprises a radiopaque material.
48. The guidewire device of clause 47, wherein the first material of the inner core comprises gold, silver, platinum, tantalum, tungsten, or an alloy thereof.
49. The guidewire device of clause 47, wherein the second material of the outer sheath comprises a cobalt-chromium alloy, a nickel-titanium alloy, a platinum alloy, a titanium alloy, or stainless steel.
50. The guidewire device of clause 49, wherein the metal composite wire comprises a fill percentage of the first material of the inner core ranging from about 10 to 45 percent along a length of the core wire.
51. The guidewire device of clause 46, wherein the first material of the inner core comprises a cobalt-chromium alloy, a nickel-titanium alloy, a platinum alloy, a titanium alloy, or stainless steel.
52. The guidewire device of clause 51, wherein the second material of the outer sheath comprises a cobalt-chromium alloy, a nickel-titanium alloy, a platinum alloy, a titanium alloy, or stainless steel.
53. The guidewire device of clause 52, wherein the metal composite wire comprises a fill percentage of the first material of the inner core ranging from about 10 to 45 percent.
54. The guidewire device of clause 46, wherein the metal composite wire comprises a fill percentage of the first material of the inner core ranging from about 10 to 45 percent.
55. The guidewire device of clause 54, wherein the fill percentage comprises a first fill percentage in the distal portion of the core wire and a second fill percentage in the proximal portion of the core wire less than the first fill percentage.
56. The guidewire device of clause 46, wherein the distal portion of the core wire further comprises a first section and a second section, and the first section comprises a constant fill percentage of the first material of the inner core, and the second section comprises a varying fill percentage of the first material of the inner core decreasing in a proximal direction.
57. The guidewire device of clause 56, wherein the first material of the inner core comprises a radiopaque material selected from the group consisting of gold, silver, platinum, tantalum, tungsten, and an alloy thereof, and the second material of the outer sheath comprises a material selected from the group consisting of a cobalt-chromium alloy, a nickel-titanium alloy, a platinum alloy, a titanium alloy, and stainless steel.
58. The guidewire device of clause 46, wherein the first material of the inner core comprises platinum, the second material of the outer sheath comprises a cobalt-chromium alloy, and the metal composite wire comprises a fill percentage of the inner core ranging from about 10 to 45 percent along a length of the core wire.
59. The guidewire device of clause 46, further comprising a tube member located near the distal portion of the core wire, the tube member comprising a plurality of cuts circumferentially extending around a central longitudinal axis of the tube member.
60. The guidewire device of clause 46, wherein the metal composite wire has an outer diameter at the proximal portion of the core wire ranging from 0.007 inches to 0.035 inches.

Those skilled in the art will appreciate that various other modifications may be made. All these or other variations and modifications are contemplated by the inventors and within the scope of the invention.

## Claims

1. A guidewire device (100) comprising:
a core wire (110, 210, 410) extending between a proximal portion (112, 212, 412) and a distal portion (114, 214, 414);
a tube member (150, 250) located near the distal portion (114, 214, 414) of the core wire (110, 210, 410), the tube member (150, 250) being secured to the core wire (110) and defining a space (120) between the core wire (110, 210, 410) and the tube member (150, 250); and
a plurality of disks (190) coupled to the distal portion (114, 214, 414) of the core wire, the plurality of disks (190) being spaced apart from each other and configured to interference-fit onto an inner surface (152) of the tube member (150, 250), wherein the plurality of disks (190) each comprises an opening (192) configured to allow the core wire (110) to pass through and align substantially with a central longitudinal axis (151) of the tube member (150).

2. The guidewire device of claim 1, wherein the plurality of disks (190) is constructed from a polymeric material.

3. The guidewire device of claim 1 or 2, wherein the opening (192) of one or more of the plurality of disks (190) is circular.

4. The guidewire device of any of the preceding claims, wherein the opening (192) of one or more of the plurality of disks (190) is a slot extending from a center to a periphery of the one or more of the plurality of disks (190).

5. The guidewire device of any of the preceding claims, wherein the tube member (150) comprises a plurality of cuts (152. 252) circumferentially extending around the central longitudinal axis (151) of the tube member, wherein preferably the plurality of cuts are vertical cuts and/or helical cuts.

6. The guidewire device of any of the preceding claims, wherein the plurality of disks (190) is fixedly secured and preferably directly secured to the core wire (110).

7. The guidewire device of any of the preceding claims, wherein the plurality of disks (190) is further fixedly secured to the tube member (150).

8. The guidewire device of any of the preceding claims, wherein the proximal portion (112) of the core wire (110) has an outer diameter equal to or greater than 0.356 mm, wherein preferably the proximal portion (112) of the core wire (110) has an outer diameter in the range between 0.356 mm and 0.965 mm.

9. The guidewire device of any of the preceding claims, comprising a radiopaque marker (260) in the tube member (250) and coupled to the core wire (210), wherein the radiopaque marker (260) comprises a radiopaque first material and a plastically deformable second material.

10. The guidewire device of claim 9, wherein the radiopaque marker is in the form of a coil wound around the distal portion of the core wire or a braid.

11. The guidewire device of claim 10, wherein the coil is formed of a metal composite wire comprising an inner core (262) of the radiopaque first material and an outer sheath (264) of the plastically deformable second material.

12. The guidewire device of claim 10 or 11, wherein the coil comprises a bifilar wire (265) comprising a first wire (266) and a second wire (268) parallel with the first wire (266), the first wire (266) of the bifilar wire comprising the radiopaque first material, and the second wire (268) of the bifilar wire comprising the plastically deformable second material, wherein preferably the plastically deformable second material comprises a nickel-cobalt alloy or stainless steel.

13. The guidewire device according to any of the preceding claims, wherein the core wire (410) comprises a metal composite wire comprising an inner core (416) of a first material and an outer sheath (418) of a second material different from the first material.

14. The guidewire device of claim 13,
wherein the first material of the inner core (416) comprises a radiopaque material, and/or
wherein the first material of the inner core (416) comprises gold, silver, platinum, tantalum, tungsten, or an alloy thereof, and/or
wherein the second material of the outer sheath (418) comprises a cobalt-chromium alloy, a nickel-titanium alloy, a platinum alloy, a titanium alloy, or stainless steel.

15. The guidewire device of claim 13 or 14, wherein the metal composite wire (410) comprises a fill percentage of the first material of the inner core (416) ranging from about 10 to 45 percent along a length of the core wire.
